# EUROPEAN PATENT SPECIFICATION

(11) **EP 2 720 635 B1**
(45) Date of publication and mention of the grant of the patent: **30.05.2018**
(21) Application number: 12731660.2
(22) Date of filing: 07.06.2012
(51) Int. Cl.: A61B 90/70, A61B 50/00, A61B 90/00

(54) **PROTECTION APPARATUS AND METHOD FOR WASHING ENDOSCOPIC MEDICAL INSTRUMENTS, SUCH AS TRANSESOPHAGEAL PROBES OR THE LIKE**
SCHUTZVORRICHTUNG UND VERFAHREN ZUM WASCHEN VON MEDIZINISCHEN ENDOSKOPISCHEN INSTRUMENTEN WIE TRANSÖSOPHAGEALE SONDEN ODER DERGLEICHEN
APPAREIL DE PROTECTION ET PROCÉDÉ DE LAVAGE D'INSTRUMENTS MÉDICAUX ENDOSCOPIQUES, TELS QUE DES SONDES TRANSSOPHAGIENNES OU ÉQUIVALENTS

(30) Priority: 14.06.2011 IT UD20110089
(43) Date of publication of application: 23.04.2014
(73) Proprietor: Steelco Spa, 31039 Riese Pio X (IT)
(72) Inventor: ZARDINI, Fabio, I-31033 Castelfranco Veneto (IT)
(74) Representative: Petraz, Davide Luigi
(86) International application number: PCT/IB2012/001096
(87) International publication number: WO 2012/172403

(56) References cited:
- EP-A1- 1 629 765
- DE-U1-202004 002 607
- US-A- 5 090 433
- US-A1- 2003 190 256
- US-A1- 2006 147 339
- US-A1- 2006 207 158

## Description

### FIELD OF THE INVENTION

The present invention concerns a protection apparatus for washing medical instruments of the tubular type made of various materials, in particular metal, plastic and/or rubber, used in medicine to explore and examine using optical apparatuses or to empty natural cavities of the organism, and introduced through natural or artificial paths. The present invention is for example applicable in cases of washing, heat-disinfection and/or chemical disinfection and drying of medical instruments of the type in question. Apart from a tubular part, called insertion tube, usually flexible and elongated which, for the purposes of the clinical examination is inserted inside the human body and has to be washed and sterilized for re-use, such instruments are also provided with electronic components, like transesophageal probes or suchlike. The electronic components are usually disposed at one end of the tubular part and, since they are very sensitive to humidity, water and other washing agents, must be protected during such treatments.

### BACKGROUND OF THE INVENTION

It is known to wash medical instruments of the endoscopic type, which get dirty during use, in a suitable washing chamber of a washer machine. Endoscopic instruments typically have a flexible tube, also called the insertion tube, able to be inserted into the patient's body, and which has a variable length and diameter, according to the applications. Inside the flexible tube, depending on the specific models of instrument, channels of various sizes and for various uses may be disposed or made, used for example for biopsies, for a video-camera inspection, for illumination, to spray air and/or water. In the washing chamber the complete washing treatment is carried out, both of the external surfaces, and of the internal surfaces and of the channels of the endoscopic instrument, using washing liquids such as water and detergent, in a first step, and using chemical disinfectant, for example with a base of glutaraldehyde or peracetic acid, in a second step.

Flexible tubes may be provided inside the washing chamber, for connection with the possible internal channels of the endoscopic instrument which, by means of a suitable watertight connector, are put in connection with devices that deliver the washing liquid, associated with the washer machine itself.

The external surface of the endoscopic instrument is treated instead by immersion in a container, for example an auxiliary tube, in the washing chamber of a suitable washer machine. The washing liquid, by means of a sleeve mounted on the auxiliary tube, is delivered inside it, to reach the whole external surface of the insertion tube.

Apart from this, the auxiliary tube also protects the insertion tube from unwanted knocks and friction during washing.

However, in the case of medical instruments of the type in question and provided with electronic components that are very sensitive to humidity, in order not to compromise the functionality of the electronic components, it is necessary to prevent any direct contact of the latter with water and humidity. This because it is highly likely that the electronic components, which are very expensive, will be damaged or will malfunction in the event of contact with water, and this is not acceptable. One peculiarity of the instruments is that it is not possible to separate the electronic components that are at one end of the insertion tube from the insertion tube itself, in order to wash them.

The problem therefore is to wash the invasive part of the medical instrument, which is inserted into the human body and which may come into contact with water, without damaging the electronic part, which is sensitive and expensive.

Usually, the need to do this entails a more laborious and inconvenient use of the washer machines as above: the insertion tube must be inserted into the washing chamber, leaving the door open while it is functioning during the washing cycle, and the electronic components must be disposed outside the chamber. However, this causes an unwanted leakage of steam, splashes of washing water, washing agents or other from the washing chamber, which dirties the surroundings of the washer machine and can also hit the operator and the electronic components and therefore, in short, it does not represent a functional, reliable and safe solution.

Document EP-A-1,629,765 describes a cleaning device for an endoscope which comprises a container having a first compartment to house a first portion of the endoscope which can be immersed in the liquid, and a second compartment to house a second portion of the endoscope that cannot be immersed in the liquid, separated from the first compartment by a wall that has a through aperture for the passage of a flexible tube which connects the first portion to the second portion of the endoscope. The container is closed at the top by a lid applied from above so as to cover the two compartments.

Document US-A-2006/0207158 describes a system for creating artificial floating birds or animals which comprises a submerged motor to animate the artificial animal. The motor is contained in a box-like container provided with a closing lid applied from above and with a through aperture for the cables of the motorization system.

Purpose of the present invention is to obtain a protection apparatus for washing endoscopic medical instruments, such as transesophageal probes or suchlike, which allows to use, safely and functionally, known washer machines, where it is possible to completely insert the instrument to be washed into the washing chamber, which can then be closed, at the same time protecting the sensitive electronic components from direct contact with the humidity and water.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purpose, an apparatus according to the present invention is used for protecting endoscopic medical instruments during washing, such as for example transesophageal probes or suchlike, having a tubular part to be washed which has at one end electronic components to be protected.

The apparatus according to the present invention comprises a closed and watertight box-like body which delimits inside it a housing compartment where the electronic components of the instrument are positioned, through an aperture, in a condition protected from the outside.

With the present invention it is therefore possible to use known washer machines safely and functionally, and to completely insert the instrument to be washed in the washing chamber, which can therefore be closed, at the same time protecting the sensitive electronic components from direct contact with humidity and water.

The present invention provides that the box-like body is provided with fluidic connection means with a connector of a pipe of a pressure generator and controller, so as to put under pressure the inside of the box-like body in the closed condition, advantageously for the purpose of verifying the watertight seal of the box-like body in the closed condition. Therefore, the solution is advantageous to verify possible losses of pressure during the whole processing cycle, which attest that the seal is not watertight and therefore the intervention of the operator is required. The box-like body is formed by at least two parts associated together, of which a first bottom part, to house the electronic components to be protected, and a second closing part, which parts are selectively mobile with respect to each other between a closed condition, in which the second part completely covers the first part protecting the electronic components inside the housing compartment, and an open condition, which allows the electronic components to be inserted inside the housing compartment. The box-like body is provided with hinging means between the first part and the second part, which allow the reciprocal rotation thereof between the closed condition and the open condition. This solution allows to open and close the apparatus extremely intuitively and quickly.

Advantageously, closing means are provided, for example the hook or snap-in type, easy and quick to use, to close the box-like body, in particular to clamp the first part with respect to the second part in the closed condition.

In some forms of embodiment, the apparatus comprises a first watertight element associated with the aperture, which allows to insert into the box-like body the tubular part that has the electronic components and also determines the watertight seal with respect to the outside.

According to a variant, the first watertight seal element is formed by a sealing member that has a through hole configured for the passage of the tubular part of the instrument which is inserted into the box-like body.

According to another variant, the sealing member is the selectively modular type and comprises a first portion, configured to connect to the aperture, and a second portion associable above the first portion to define the through hole.

In one form of embodiment, both the first portion and the second portion of the sealing member have facing mating semi-seatings that define the through hole.

In some forms of embodiment, the first part and the second part cooperate reciprocally in the closed condition along mating peripheral closing edges able to be positioned adjacent to each other so as to close the box-like body, a second watertight seal element being positioned along said closing edges. In variant solutions, each of the closing edges has a peripheral hollow of a shape mating with the second watertight seal element.

According to a variant, a head end of the second portion of the sealing member has a segment with a hollow mating in shape and size with a corresponding one of the hollows, which in use is aligned with the latter. In this way, it is possible to cause a continuous support of the second watertight element even in the zone of the aperture where the sealing member is positioned, thus guaranteeing the watertight seal.

The present invention also concerns a protection method for washing endoscopic medical instruments, such as transesophageal probes or suchlike, having a tubular part to be subjected to washing which at one end has electronic components to be protected. The method provides to position, in a condition protected from the outside, the electronic components of the instrument inside a housing compartment of a box-like body closed with a watertight seal. The method according to the present invention provides a step of verifying the watertight seal of the box-like body, in which the inside of the closed box-like body is put under pressure and any possible leakage from the box-like body is evaluated according to how the measurement of the overpressure inside the box-like body develops. In particular, the invention provides to measure the pressure generated inside the box-like body and to identify a possible lowering of pressure, thus correlating it to the lack of watertight seal of the closed box-like body. This last condition is to be avoided, for the application of washing medical instruments in question, and an indication to that effect, before starting washing, is therefore useful so as to make the necessary provisions and to prevent the sensitive electronic components of the medical instrument from entering into contact with the washing fluid in the course of the washing.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a perspective view of an apparatus according to the present invention in a closed condition;
- fig. 2 is a front view of the apparatus in fig. 1;
- fig. 3 is a lateral view of the apparatus in fig. 1;
- fig. 4 is a perspective view in separate parts of the apparatus in fig. 1;
- fig. 5 is a perspective view of an apparatus according to the present invention in an open condition.

To facilitate comprehension, the same reference numbers have been used, where possible, to identify identical common elements in the drawings. It is understood that elements and characteristics of one form of embodiment can conveniently be incorporated into other forms of embodiment without further clarifications.

### DETAILED DESCRIPTION OF A PREFERENTIAL FORM OF EMBODIMENT

With reference to the attached drawings, an apparatus 10 according to the present invention is used to protect electronic components of a tubular medical instrument, in particular the endoscopic type, such as a transesophageal probe, schematized partly with dashes and indicated by the reference number 12, in the course of a washing, heat-disinfection and drying operation performed inside a washing chamber of a suitable washer machine. The instrument 12 is generally formed by an insertion tube and by electronic components located at one end of it.

The apparatus 10 according to the present invention comprises a watertight box-like body 14 that delimits internally a housing compartment 15 to position, in a condition protected from the outside, the electronic components of the instrument 12 as above, and which typically cannot be separated from the insertion tube of the instrument 12 to be washed.

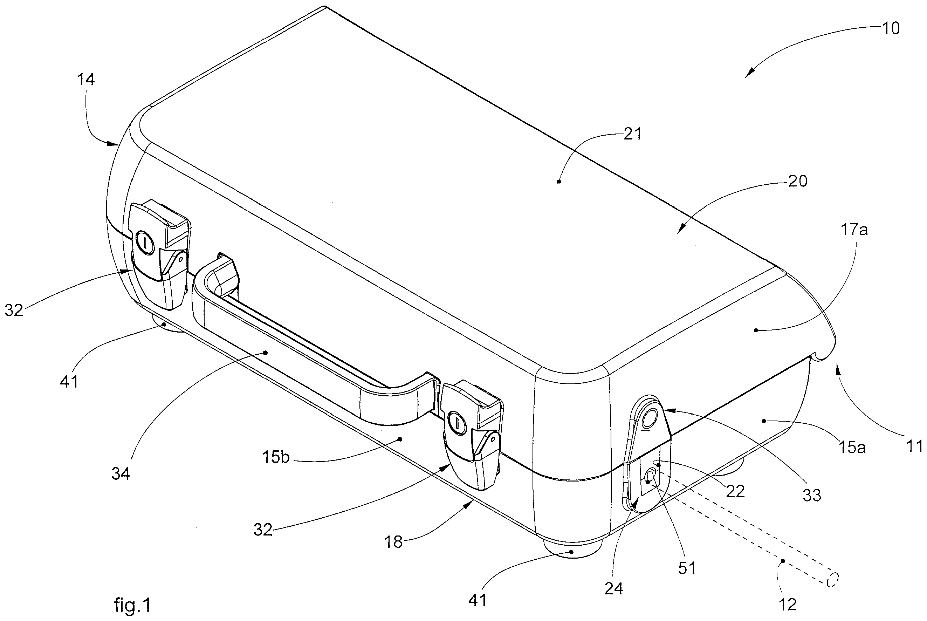

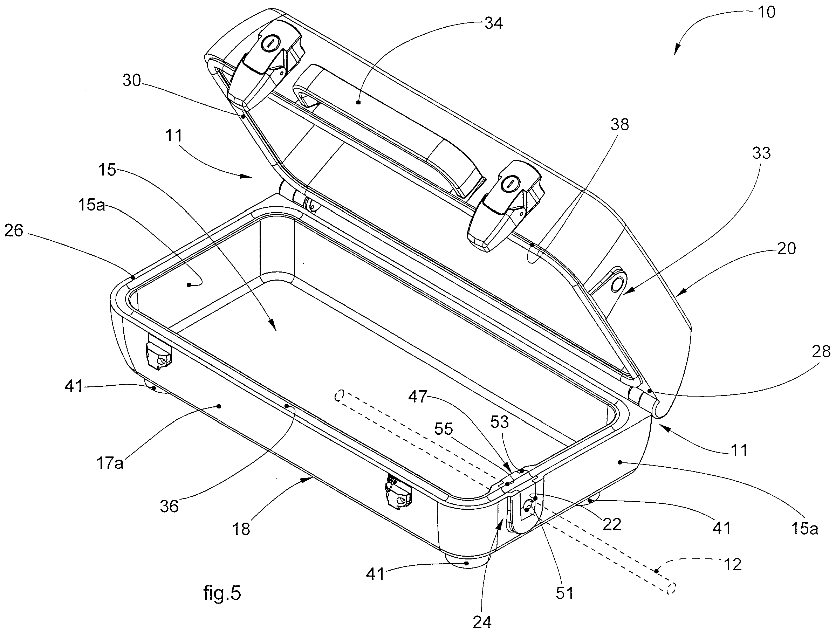

The box-like body 14 is formed by at least two parts, associated together, of which a first bottom part 18, to house the part of the electronic components to be protected, and a second closing part 20, which parts are selectively mobile with respect to each other between a closed condition, in which the second part 20 completely covers the first part 18 protecting the electronic components inside the housing compartment 15 (fig. 1), and an open condition, which allows the electronic components to be inserted, together with the tubular part of the instrument 12 connected therewith, inside the housing compartment 15 (fig. 5).

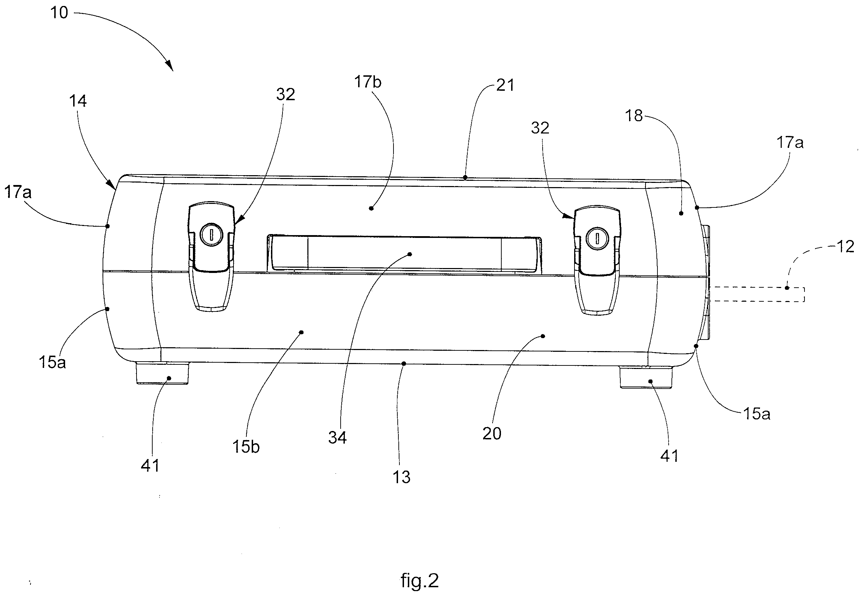

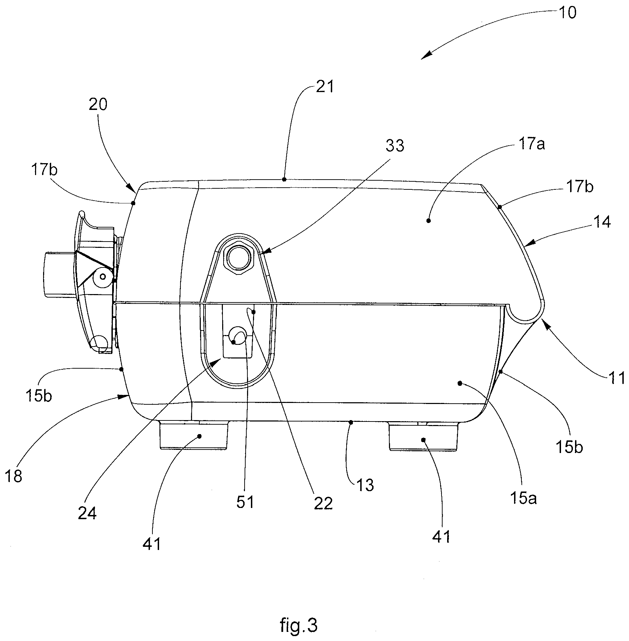

In this case, the first part 18 and the second part 20 are defined by corresponding short lateral walls 15a, 17a and long lateral walls 15b, 17b and by corresponding base walls 13, 21, which in this case function as a bottom wall and top wall (figs. 2 and 3).

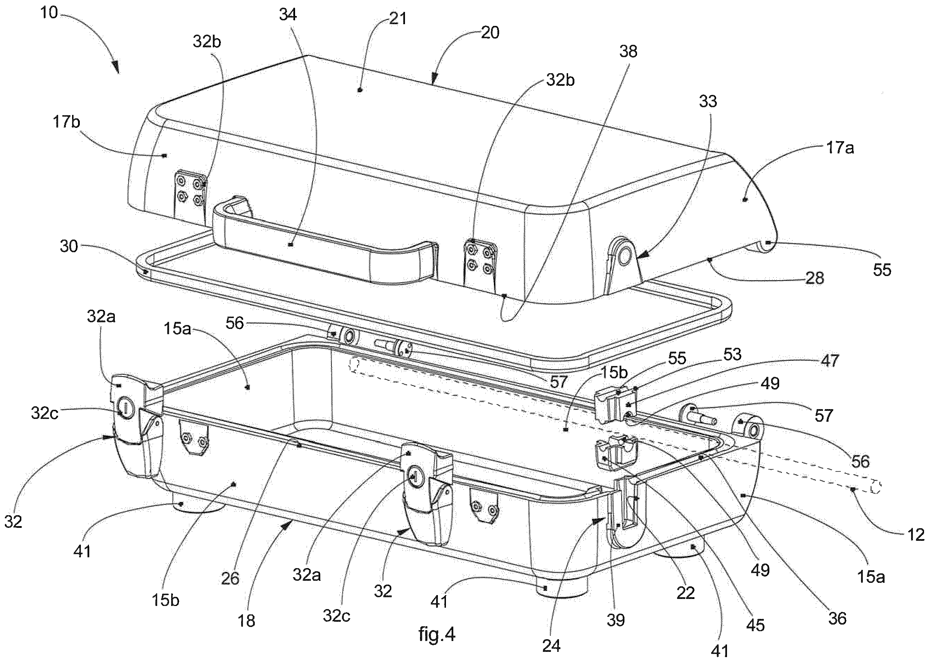

According to one form of embodiment, the box-like body 14 is provided with hinging means 11 between the first part 18 and the second part 20, which allow the reciprocal rotation thereof between the closed condition and the open condition. In the solution shown, the hinging means 11 comprise respective articulated portions 56 pivoted by means of pins or screws 57 (fig. 4).

Furthermore, in some forms of embodiment supporting feet 41 may be provided, connected to the first part 18 that functions as a supporting bottom of the apparatus 10.

In some forms of embodiment the box-like body is a parallelepiped with a rectangular, square or generally quadrangular base, advantageously with beveled corners, although another shape could be provided, such as for example cylindrical, oval or other, according to needs. The box-like body 14 can be made of a material suitable to support the conditions of temperature and humidity and the presence of possible detergent agents inside the washing chamber, for example made of plastic, such as plastic, like polyurethane.

According to the form of embodiment shown in the attached drawings, the box-like body 14 is conformed as a portable case and the first part 18 and the second part 20 are defined substantially by two symmetrical semi-shells, hinged to each other and selectively mobile between the closed and open conditions: a first semi-shell supports and houses the electronic components of the instrument 12 and a second semi-shell acts as a closing lid.

The box-like body 14 also has an aperture 22 (fig. 4), in this case made through the first part 18, for the insertion and passage of a part of the instrument 12 connected to the electronic components, the aperture 22 being associated with a first watertight seal element 24. Advantageously, it is the insertion of the tubular part of the instrument 12 through the aperture 22 that, in cooperation with the first watertight seal element 24, determines the watertight closure of the aperture 22 when the apparatus 10 is in its closed condition.

In this case, the aperture is concave, with an open profile, that is, its section opens through the peripheral edge 26 of the first part 18, so as to allow the tubular part of the instrument 12 to be positioned straddling the peripheral edge 26, that is, by inserting it from above and not transversely, without interfering with other parts of the apparatus 10. For example the aperture 22 is U-shaped, made along the height of the first part 18, in particular in this case through one of the short lateral walls 15a.

The aperture in this case is peripherally delimited by a concave connection portion 49, having a desired geometry suitable for connection with the first watertight seal element 24.

In this case, the first watertight seal element 24 is formed by a modular sealing member 43 associable with the connection portion 49, through which the tubular part of the instrument 12 that is inserted into the box-like body 14 passes.

The sealing member 43 comprises a first portion 45, configured to connect to the connection portion 39, and a second portion 47 which couples with the first portion 45. For example, the first portion 45 and the second portion 47 are made of rubber.

The first portion 45 and the second portion 47 are shaped in a mating fashion, so that once they are coupled together, by interposing the tubular part of the instrument 12 inserted with its electronic components into the box-like body 14, a housing through hole 51 is defined (fig. 3), which allows to position the tubular part of the instrument 12 straddling the first part 18.

In particular, it is provided that both the first portion 45 and the second portion 47 of the sealing member 43 have facing mating semi-seatings 49 that, once the first portion 45 and the second portion 47 have been connected, define the through hole 51, with a shape and size mating with that of the tubular part of the instrument 12 that is made to pass through the aperture 22, so that, when the apparatus 10 is in its closed condition, there is a substantial watertight seal through the through hole 51 as well. In substance, the sealing member 43 acts as a fairlead element, to allow the tubular part of the instrument 12 carrying the electronic components to be protected during washing to be inserted inside the box-like body 14, guaranteeing the watertight seal. In particular, the closure compression of the second part 20 on the first part 18 determines the close adherence of the sealing member 43 around the tubular part of the instrument 12, obtaining the desired watertight seal.

The first part 18 and the second part 20 cooperate reciprocally in the closed condition along mating peripheral closing edges 26, 28, able to be positioned adjacent to each other so as to close the box-like body 14.

According to the present invention, moreover, a second watertight seal element 30 is provided, located along the closing edges 26, 28 of the first part 18 and the second part 20 which, in the closed condition, has the function of determining the watertight seal inside the box-like body 14.

In some forms of embodiment, the second watertight seal element 30 is a closed annular packing, generally made of rubber, silicone or similar material suitable for the purpose, with shape and size substantially the same as those of the closing edges 26, 28. The closing of the second part 20 on the first part 18 determines the compression of the second watertight seal element 30 which is placed between them, thus determining the desired sealing effect.

Advantageously, each of the closing edges 26, 28 has a peripheral hollow 36, 38 (figs. 4, 5), in this case continuous, of a shape mating with the second watertight seal element 30. In this way, when the apparatus 10 is in the open condition, the second watertight seal element 30 can be inserted into one or the other hollow 36, 38, protruding toward the outside by a determinate height; in the case of fig. 5 it is positioned in the hollow 38 and then, passing to the closed condition as in fig. 1, it is inserted with its protruding part into the mating hollow, in this case hollow 36, and is compressed between the first part 18 and the second part 20.

Advantageously, moreover, the head end 53 of the second portion 47 of the sealing member 43 has a segment with a hollow 55 mating in shape and size with the hollow 36 or 38 of the first part 18 or second part 20, depending on where the aperture 22 with the open profile is made. The segment with the hollow 65 is correctly aligned, when the sealing member 43 is composed, with the corresponding hollow 36, 38, so as to compensate the discontinuity along the peripheral edge given by the presence of the open-profile aperture 22 made along the wall of the box-like body. It is thus possible to correctly house, in the segment with the hollow 65, a corresponding part of the second watertight seal element 30 so as to guarantee, in this zone too, a uniform and continuous seal over the whole perimeter of the box-like body 14 in the closed condition.

Advantageously, given the same size of the aperture 22, it may be possible to provide a series or kit of sealing members 43 of different conformations and sizes, in particular with regard to the diameter of the through hole 51; these may be selectively associated with the aperture 22, and are interchangeable depending on the sizes of the tubular part of the instrument 12 which on each occasion has to pass through the box-like body 14.

According to one form of embodiment, the box-like body 14 comprises closing means 32 to reciprocally clamp the first part 18 and second part 20 in the closed condition. In particular, the closing means 32, providing a hook portion 32a on the first part 18 and an attachment portion 32b on the second part 20, act by traction of the second part 20 toward the first part 18, obtaining the desired compression of the first watertight seal element 24, and also the second watertight seal element 30 (fig. 4). Advantageously, the closing means 32 may be provided with a lock 32c with a key, so that they can be opened only by authorized operators (fig. 4).

According to one form of embodiment, the box-like body 14 comprises fluidic connection means 33 for insertion and rapid connection with a connector provided at the end of a pipe, not shown in the drawings, of a pressure generator and controller, so that, in the closed condition, it is possible to put the inside of the box-like body 14 under pressure. The fluidic connection means 33 are advantageously configured to resist the counter-pressure deriving from the internal pressure created in the box-like body 14.

According to one form of embodiment, the box-like body 14 is of a size so that it is easily portable and manageable and, as shown in the attached drawings, may provide an external handle 34 that facilitates transport (fig. 2).

According to a variant, at the sides of the external handle 34, closing means 32 are provided, in this case formed by a snap-in or hook closing mechanism of a known type.

In some variant forms of embodiment, several apertures 22 may be provided, made as described above, and associated with similar first watertight seal elements 24, to allow to wash several instruments of the type in question simultaneously, in conditions where the corresponding electronic components are protected from humidity.

The apparatus 10 is used as follows. In particular, the apparatus 10 is put in the open condition of fig. 5, the part of electronic components of the instrument 12 is inserted into the housing compartment 15, part of the insertion tube is housed in the aperture 22 and the apparatus 10 is put in the closed condition (fig. 1). In particular, the method provides to house the tubular part of the instrument 12 carrying the electronic components astride the first portion 45, in the corresponding semi-seating 49, and subsequently the tubular part is clamped by the second portion 47 that is coupled above, thus housing the tubular part in the through hole 51 which is defined. The closing of the second part 20, rotated on the first part 18, determines the cooperation of the second watertight seal element 30 with the closing edges 26, 28 and the corresponding hollows 36, 38, and also with the segment with the hollow 65 of the sealing member 43, so as to have a substantial watertight seal on the whole closed perimeter of the box-like body 14.

Subsequently, the instrument 12 thus associated with the apparatus 10 in the washing chamber where the required process is carried out. At this point the apparatus contains and protects the electronic components in the box-like body 14. The closing of the box-like body 14 determines the cooperation of the second watertight seal element 30 with the first part 18 and the second part 20, and also of the first watertight seal element 24 with the tubular part of the instrument 12 passing through the aperture 22.

Advantageously, the conformation described above of the aperture 22 and the first watertight seal element 24 allows to position the part of the instrument 12 that has to pass through the box-like body 14 astride the sealing member 43, passing over the peripheral edges 26, 28, when the apparatus 10 is in the open condition (fig. 1). This is advantageous because it limits or prevents contact and friction of the part of the instrument 12 to be washed against the walls delimiting the aperture 22 and in general against the box-like body 14, thus reducing the possibility of dirt or pollution on the walls of the apparatus 10.

Advantageously, a step is provided to verify the watertight seal of the apparatus 10 in the closed condition, preferably before it is inserted into the washing chamber, or in any case before the washing process is started. The verification is carried out by the operator by connecting a pressure generator to the connection means 33 of the apparatus 10 in the closed condition and by putting the internal housing compartment 15, which contains the electronic components to be protected, under pressure, at a desired value of internal super-pressure, for example about 200 mbar. If the closed condition is not guaranteed with a watertight seal, or if the first watertight seal element 24 and/or the second watertight seal element 30, or in general the first part 18 and the second part 20, are not correctly coupled, a loss of pressure is obtained, or in any case the desired value of super-pressure is not reached by the pressure generator which, thanks to its own pressure sensor, will be able to signal the anomaly, by means of a visual or acoustic alarm, thus allowing the operator to verify the watertight seal and to intervene in order to restore it before starting the washing.

The operation to control and verify the pressure, and hence the watertight seal, may advantageously be performed during the whole duration of the process, monitoring the development of the measurement of the pressure values in the closed internal housing compartment. The generation of the desired pressure, and the verification of the stability thereof, are advantageously carried out automatically, thanks to the connection with the machine to generate and control the pressure by means of the connection means 33.

## Claims

1. Protection apparatus for washing endoscopic medical instruments (12), such as transesophageal probes or suchlike, having a tubular part to be subjected to washing which at one end has electronic components to be protected, comprising a watertight box-like body (14) that delimits internally a housing compartment (15) to position, through an aperture (22), the electronic components of the instrument (12) in a protected condition from the outside, said box-like body (14) being **characterized in** comprising fluidic connection means (33) with a connector of a pipe of a pressure generator and controller, so as to put under pressure the inside of the box-like body (14) in the closed condition, wherein the box-like body (14) is formed by two symmetrical semi-shells parts, hinged to each other, of which a first bottom part (18), to house the electronic components to be protected, and a second closing part (20), which parts are selectively mobile with respect to each other between a closed condition, in which the second part (20) completely covers the first part (18) protecting the electronic components inside the housing compartment (15), and an open condition, which allows said electronic components to be inserted inside the housing compartment (15).

2. Apparatus as in claim 1, **characterized in that** the box-like body (14) is provided with hinging means (11) between the first part (18) and the second part (20), which allow the reciprocal rotation thereof between the closed condition and the open condition.

3. Apparatus as in claim 1 or 2, **characterized in that** it comprises a first watertight element (24) associated with the aperture (22), which allows the insertion into the box-like body (14) of the tubular part of the instrument (12) that has said electronic components and also determines the watertight seal with respect to the outside.

4. Apparatus as in claim 3, **characterized in that** the first watertight seal element (24) is formed by a sealing member (43) that has a through hole (51) configured for the passage of the tubular part of the instrument (12) which is inserted into the box-like body (14).

5. Apparatus as in claim 4, **characterized in that** said sealing member (43) is the selectively modular type and comprises a first portion (45), configured to connect to the aperture (22), and a second portion (47) associable above the first portion (45) to define the through hole (51).

6. Apparatus as in claim 5, **characterized in that** both the first portion (45) and the second portion (47) of the sealing member (43) have facing mating semi-seatings (49) that define said through hole (51).

7. Apparatus as in claims 4, 5 or 6, **characterized in that** the aperture (22) and the first watertight seal element (24) are configured to allow to position a part of the instrument (12) that has to pass through the box-like body (14) astride the sealing member (43), passing over mating peripheral edges (26, 28) of the first part (18) and second part (20), when the box-like body (14) is in the open condition.

8. Apparatus as in claim 1 or 2, **characterized in that** the first part (18) and the second part (20) cooperate reciprocally in the closed condition along mating peripheral closing edges (26, 28) able to be positioned adjacent to each other so as to close the box-like body (14), a second watertight seal element (30) being positioned along said closing edges (26, 28).

9. Apparatus as in claims 8, **characterized in that** each of the closing edges (26, 28) has a peripheral hollow (36, 38) of a shape mating with the second watertight seal element (30).

10. Apparatus as in claim 5 and 9, **characterized in that** a head end (53) of the second portion (47) of the sealing member (43) has a segment with a hollow (55) mating in shape and size with a corresponding one of said hollows (36, 38).

11. Apparatus as in any claim hereinbefore, **characterized in that** the box-like body (14) is conformed as a portable case.

12. Apparatus as in any claims hereinbefore, **characterized in that** the box-like body (14) comprises closing means (32) to reciprocally clamp the first part (18) and second part (20) in the closed condition.

13. Apparatus as in claim 12, **characterized in that** the closing means (32) are provided with a hook portion (32a) on the first part (18) and an attachment portion (32b) on the second part (20), and are configured to act by traction of the second part (20) toward the first part (18).

14. Protection method for washing endoscopic medical instruments (12), such as transesophageal probes or suchlike, having a tubular part to be subjected to washing which at one end has electronic components to be protected, comprising providing a box-like body (14) that delimits internally a housing compartment (15) to position, through an aperture (22), the electronic components of the instrument (12) in a protected condition from the outside, wherein said box-like body (14) is formed by two symmetrical semi-shells parts, hinged to each other, of which a first bottom part (18), to house the electronic components to be protected, and a second closing part (20), which parts are selectively mobile with respect to each other between a closed condition, in which the second part (20) completely covers the first part (18) protecting the electronic components inside the housing compartment (15), and an open condition, which allows said electronic components to be inserted inside the housing compartment (15),
moving the first bottom part (18) and the second closing part (20) relative to each other to provide the box-like body (14) in said open condition;
positioning, in a condition protected from the outside, the electronic components of the instrument (12) inside said housing compartment (15) of said box-like body (14) closed with a watertight seal,
moving the first bottom part (18) and the second closing part (20) relative to each other to provide the box-like body (14) in said closed condition;
verifying the watertight seal of the box-like body (14), putting the inside of the closed box-like body (14) under pressure and evaluating any possible leakage from the box-like body (14) according to the development of the measurement of the overpressure inside the box-like body (14).

15. Protection method as in claim 14, **characterized in that** it comprises positioning a part of the instrument (12) that has to pass through the box-like body (14) astride a sealing member (43) of a first watertight element (24) associated with the aperture (22), passing over mating peripheral edges (26, 28) of the first part (18) and second part (20), when the box-like body (14) is in the open condition.

## Patentansprüche

1. Schutzvorrichtung zum Waschen von endoskopischen medizinischen Instrumenten (12) wie transösophagealen Sonden oder dergleichen, die ein rohrförmiges Teil aufweisen, das Waschen unterzogen werden soll und an einem Ende elektronische Komponenten aufweist, die geschützt werden sollen, umfassend einen wasserdichten kastenförmigen Körper (14), der innen ein Aufnahmefach (15) begrenzt, um, durch eine Öffnung (22), die elektronischen Komponenten des Instrumentes (12) in einem von außen geschützten Zustand zu positionieren, wobei der kastenförmige Körper (14) **dadurch gekennzeichnet ist, dass** er fluidische Verbindungsmittel (33) mit einem Verbinder eines Rohres eines Druckgenerators und -reglers umfasst, so dass das Innere des kastenförmigen Körpers (14) im geschlossenen Zustand unter Druck gesetzt wird, worin der kastenförmige Körper (14) durch zwei symmetrische, aneinander angelenkte, Halbschalenteile gebildet ist, von denen es sich um ein erstes Unterteil (18), um die zu schützenden elektronischen Komponenten unterzubringen, und um ein zweites Verschlussteil (20) handelt, wobei diese Teile selektiv zwischen einem geschlossenen Zustand, in dem das zweite Teil (20) das erste Teil (18) völlig abdeckt, das die elektronischen Komponenten innerhalb des Aufnahmefachs (15) schützt, und einem offenen Zustand, der den elektronischen Komponenten ermöglicht, in das Aufnahmefach (15) hinein eingeführt zu werden, zueinander beweglich sind.

2. Vorrichtung nach Anspruch 1, **dadurch gekennzeichnet, dass** der kastenförmige Körper (14) mit Scharniereinrichtungen (11) zwischen dem ersten Teil (18) und dem zweiten Teil (20) versehen ist, die die gegenseitige Drehung derselben zwischen dem geschlossenen Zustand und dem offenen Zustand ermöglichen.

3. Vorrichtung nach Anspruch 1 oder 2, **dadurch gekennzeichnet, dass** sie ein erstes wasserdichtes Element (24) umfasst, das mit der Öffnung (22) verbunden ist, die die Einführung des rohrförmigen Teils des Instrumentes (12), das die elektronischen Komponenten aufweist, in den kastenförmigen Körper (14) hinein ermöglicht und auch die wasserdichte Abdichtung gegenüber dem Äußeren bestimmt.

4. Vorrichtung nach Anspruch 3, **dadurch gekennzeichnet, dass** das erste wasserdichte Abdichtungselement (24) von einem Abdichtungsteil (43) gebildet ist, das ein Durchgangsloch (51) aufweist, das für den Durchgang des rohrförmigen Teils des Instrumentes (12) eingerichtet ist, das in den kastenförmigen Körper (14) eingeführt ist.

5. Vorrichtung nach Anspruch 4, **dadurch gekennzeichnet, dass** das Abdichtungsteil (43) vom selektiv modularen Typ ist und einen ersten Abschnitt (45), der dafür eingerichtet ist, um sich mit der Öffnung (22) zu verbinden, und einen zweiten Abschnitt (47) umfasst, der über dem ersten Abschnitt (45) verbindbar ist, um das Durchgangsloch (51) zu definieren.

6. Vorrichtung nach Anspruch 5, **dadurch gekennzeichnet, dass** sowohl der erste Abschnitt (45) als auch der zweite Abschnitt (47) des Abdichtungsteils (43) gegenüberstehende zueinander passende Halbsitze (49) aufweisen, die das Durchgangsloch (51) definieren.

7. Vorrichtung nach den Ansprüchen 4, 5 oder 6, **dadurch gekennzeichnet, dass** die Öffnung (22) und das erste wasserdichte Abdichtungselement (24) dafür eingerichtet sind, um zu ermöglichen, ein Teil des Instrumentes (12) zu positionieren, das den kastenförmigen Körper (14) rittlings auf dem Abdichtungsteil (43) durchzulaufen hat, indem es zueinander passende Umfangskanten (26, 28) des ersten Teils (18) und des zweiten Teils (20) überschreitet, wenn der kastenförmige Körper (14) sich im offenen Zustand befindet.

8. Vorrichtung nach Anspruch 1 oder 2, **dadurch gekennzeichnet, dass** das erste Teil (18) und das zweite Teil (20) wechselseitig im geschlossenen Zustand entlang zueinander passender Umfangsverschlusskanten (26, 28) zusammenwirken, die aneinander angrenzend positioniert werden können, um den kastenförmigen Körper (14) zu schließen, wobei ein zweites wasserdichtes Abdichtungselement (30) entlang der Umfangsverschlusskanten (26, 28) positioniert ist.

9. Vorrichtung nach Anspruch 8, **dadurch gekennzeichnet, dass** jede der Verschlusskanten (26, 28) einen Umfangshohlraum (36, 38) aufweist, dessen Gestalt zum zweiten wasserdichten Abdichtungselement (30) passt.

10. Vorrichtung nach Anspruch 5 und 9, **dadurch gekennzeichnet, dass** ein Kopfende (53) des zweiten Abschnitts (47) des Abdichtungsteils (43) ein Segment mit einem Hohlraum (55) aufweist, dessen Gestalt und Größe zu jeweils einem der Hohlräume (36, 38) passen.

11. Vorrichtung nach einem der vorangehenden Ansprüche, **dadurch gekennzeichnet, dass** der kastenförmige Körper (14) wie ein tragbarer Koffer ausgestaltet ist.

12. Vorrichtung nach einem der vorangehenden Ansprüche, **dadurch gekennzeichnet, dass** der kastenförmige Körper (14) Verschlussmittel (32) umfasst, um das erste Teil (18) und das zweite Teil (20) im geschlossenen Zustand gegenseitig zu klemmen.

13. Vorrichtung nach Anspruch 12, **dadurch gekennzeichnet, dass** die Verschlussmittel (32) mit einem Hakenabschnitt (32a) am ersten Teil (18) und mit einem Befestigungsabschnitt (32b) am zweiten Teil (20) versehen sind und dafür eingerichtet sind, um durch Zug des zweiten Teils (20) in Richtung auf das erste Teil (18) zu wirken.

14. Schutzverfahren zum Waschen von endoskopischen medizinischen Instrumenten (12) wie transösophagealen Sonden oder dergleichen, die ein rohrförmiges Teil aufweisen, das Waschen unterzogen werden soll und an einem Ende elektronische Komponenten aufweist, die geschützt werden sollen, umfassend:
Vorsehen eines kastenförmigen Körpers (14), der innen ein Aufnahmefach (15) begrenzt, um, durch eine Öffnung (22), die elektronischen Komponenten des Instrumentes (12) in einem von außen geschützten Zustand zu positionieren, wobei der kastenförmige Körper (14) durch zwei symmetrische, aneinander angelenkte, Halbschalenteile gebildet ist, von denen es sich um ein erstes Unterteil (18), um die zu schützenden elektronischen Komponenten unterzubringen, und um ein zweites Verschlussteil (20) handelt, wobei diese Teile selektiv zwischen einem geschlossenen Zustand, in dem das zweite Teil (20) das erste Teil (18) völlig abdeckt, das die elektronischen Komponenten innerhalb des Aufnahmefachs (15) schützt, und einem offenen Zustand, der den elektronischen Komponenten ermöglicht, in das Aufnahmefach (15) hinein eingeführt zu werden, zueinander beweglich sind,
Bewegen des ersten Unterteils (18) und des zweiten Verschlussteils (20) relativ zueinander, um den kastenförmigen Körper (14) im offenen Zustand bereitzustellen;
Positionieren, in einem von außen geschützten Zustand, der elektronischen Komponenten des Instrumentes (12) innerhalb des Aufnahmefachs (15) des kastenförmigen Körpers (14), der mit einer wasserdichten Abdichtung geschlossen ist,
Bewegen des ersten Unterteils (18) und des zweiten Verschlussteils (20) relativ zueinander, um den kastenförmigen Körper (14) im geschlossenen Zustand bereitzustellen;
Überprüfen der wasserdichten Abdichtung des kastenförmigen Körpers (14), indem das Innere des geschlossenen kastenförmigen Körpers (14) unter Druck gesetzt und jeder mögliche Austritt aus dem kastenförmigen Körper (14) aufgrund des Verlaufs der Messung des Überdrucks innerhalb des kastenförmigen Körpers (14) eingeschätzt wird.

15. Schutzverfahren nach Anspruch 14, **dadurch gekennzeichnet, dass** es das Positionieren eines Teils des Instrumentes (12) umfasst, das den kastenförmigen Körper (14) rittlings auf einem Abdichtungsteil (43) eines ersten wasserdichten Elementes (24), das mit der Öffnung (22) verbunden ist, durchzulaufen hat, indem es zueinander passende Umfangskanten (26, 28) des ersten Teils (18) und des zweiten Teils (20) überschreitet, wenn der kastenförmige Körper (14) sich im offenen Zustand befindet.

## Revendications

1. Appareil de protection pour le lavage d'instruments médicaux d'endoscopie (12), tels que des sondes transoesophagiennes ou similaires, ayant une partie tubulaire destinée à être soumise à un lavage et qui comporte à une extrémité des composants électroniques devant être protégés, comprenant un corps en forme boîte étanche à l'eau (14) qui délimite l'intérieur d'un compartiment de logement (15) pour positionner, à travers une ouverture (22), les composants électroniques de l'instrument (12) dans un état protégé de l'extérieur, ledit corps en forme de boîte (14) étant **caractérisé en ce qu'il** comprend des moyens de connexion fluidiques (33) avec un connecteur d'un tuyau d'un générateur de pression et un dispositif de commande, de manière à mettre sous pression l'intérieur du corps en forme de boîte (14) à l'état fermé, le corps en forme de boîte (14) étant formé par deux parties en forme de demi-coquilles symétriques, articulées l'une à l'autre, dont une première partie inférieure (18) pour loger les composants électroniques à protéger et une seconde partie de fermeture (20), ces parties étant sélectivement mobiles l'une par rapport à l'autre, entre un état fermé, dans lequel la seconde partie (20) couvre complètement la première partie (18) en protégeant les composants électroniques à l'intérieur du compartiment de logement (15) et un état ouvert, qui permet auxdits composants électroniques d'être introduits à l'intérieur du compartiment de logement (15).

2. Appareil selon la revendication 1, **caractérisé en ce que** ledit corps en forme de boîte (14) est pourvu de moyens d'articulation (11) entre la première partie (18) et la seconde partie (20), ce qui permet à ces dernières de tourner l'une par rapport à l'autre entre l'état fermé et l'état ouvert.

3. Appareil selon la revendication 1 ou 2, **caractérisé en ce qu'il** comprend un premier élément étanche à l'eau (24) associé à l'ouverture (22) qui permet l'introduction dans le corps en forme de boîte (14) de la partie tubulaire de l'instrument (12) qui comporte lesdits composants électroniques et détermine aussi l'étanchéité à l'eau par rapport à l'extérieur.

4. Appareil selon la revendication 3, **caractérisé en ce que** le premier élément d'étanchéité à l'eau (24) est formé par un organe d'étanchéité (43) qui comporte un trou traversant (51) configuré pour le passage de la partie tubulaire de l'instrument (12) qui est introduite dans le corps en forme de boîte (14).

5. Appareil selon la revendication 4, **caractérisé en ce que** ledit organe d'étanchéité (43) est du type modulable sélectivement et comprend une première partie (45), configurée pour être connectée à l'ouverture (22), et une seconde partie (47) associée au-dessus de la première partie (45) pour définir le trou traversant (51).

6. Appareil selon la revendication 5, **caractérisé en ce que** la première partie (45) ainsi que la seconde partie (47) de l'organe d'étanchéité (43) comportent des demi-embases correspondantes opposées (49) qui définissent ledit trou traversant (51).

7. Appareil selon les revendications 4, 5 ou 6, **caractérisé en ce que** l'ouverture (22) et le premier élément d'étanchéité à l'eau (24) sont configurés pour permettre le positionnement d'une partie de l'instrument (12) qui doit passer à travers le corps en forme de boîte (14) à cheval sur l'organe d'étanchéité (43), en passant au-dessus de bords périphériques correspondants (26, 28) de la première partie (18) et de la seconde partie (20), lorsque le corps en forme de boîte (14) est à l'état ouvert.

8. Appareil selon la revendication 1 ou 2, **caractérisé en ce que** la première partie (18) et la seconde partie (20) coopèrent l'une avec l'autre à l'état fermé le long de bords de fermeture périphériques correspondants (26, 28) aptes à être positionnés l'un à côté de l'autre de manière à fermer le corps en forme de boîte (14), un second élément d'étanchéité à l'eau (30) étant positionné le long desdits bords de fermeture (26, 28).

9. Appareil selon la revendication 8, **caractérisé en ce que** chacun des bords de fermeture (26, 28) a un creux périphérique (36, 38) ayant une forme correspondant avec le second élément d'étanchéité à l'eau (30).

10. Appareil selon les revendications 5 et 9, **caractérisé en ce qu'**une extrémité de tête (53) de la seconde partie (47) de l'organe d'étanchéité (43) a un segment avec un creux (55) correspondant en forme et en taille avec l'un des trous (36, 38).

11. Appareil selon l'une des revendications précédentes, **caractérisé en ce que** le corps en forme de boîte (14) est conformé en tant que boîtier portable.

12. Appareil selon l'une des revendications précédentes, **caractérisé en ce que** le corps en forme de boîte (14) comprend des moyens de fermeture (32) pour serrer l'une par rapport à l'autre la première partie (18) et la seconde partie (20) à l'état fermé.

13. Appareil selon la revendication 12, **caractérisé en ce que** les moyens de fermeture (32) sont pourvus d'une partie formant crochet (32a) sur la première partie (18) et d'une partie de fixation (32b) sur la seconde partie (20), et sont configurés pour agir par traction de la seconde partie (20) vers la première partie (18).

14. Procédé de protection pour le lavage d'instruments médicaux endoscopiques (12), tels que des sondes transoesophagiennes ou similaires, ayant une partie tubulaire destinée à être soumise à un lavage et ayant à une extrémité des composant électroniques devant être protégés, comprenant :
- la mise à disposition d'un corps en forme de boîte (14) qui délimite l'intérieur d'un compartiment de logement (15) pour positionner, à travers une ouverture (22), les composants électroniques de l'instrument (12) à l'état protégé de l'extérieur, le corps en forme de boîte (14) étant formé de deux parties en demi-coquille symétriques, articulées l'une à l'autre, dont une première partie inférieure (18) pour loger les composants électroniques à protéger et une seconde partie de fermeture (20), ces parties étant mobiles sélectivement l'une par rapport à l'autre entre un étant fermé, dans lequel la seconde partie (20) couvre complètement la première partie (18) protégeant les composants électroniques à l'intérieur du compartiment de logement (15), et un état ouvert qui permet auxdits composants électroniques d'être introduits à l'intérieur du compartiment de logement (15),
- le déplacement de la première partie inférieure (18) et de la seconde partie inférieure (20) l'une par rapport à l'autre pour mettre ledit corps en forme de boîte (14) dans ledit état ouvert ;
- le positionnement, dans un état protégé de l'extérieur, des composants électroniques de l'instrument (12) à l'intérieur dudit compartiment de logement (15) dudit corps en forme de boîte (14) fermé de manière étanche à l'eau,
- le déplacement de la première partie inférieure (18) et de la seconde partie de fermeture (20) l'une par rapport à l'autre pour mettre le corps en forme de boîte (14) dans ledit état fermé ;
- la vérification de l'étanchéité à l'eau du corps en forme de boîte (14), mettre l'intérieur du corps en forme de boîte fermé (14) sous pression et évaluer toute fuite possible depuis le corps en forme de boîte (14) en fonction de l'évolution de la mesure de la surpression à l'intérieur du corps en forme de boîte (14).

15. Procédé de protection selon la revendication 14, **caractérisé en ce qu'il** comprend le positionnement d'une partie de l'instrument (12) qui doit passer à travers le corps en forme de boîte (14) à cheval sur un organe d'étanchéité (43) d'un premier élément étanche à l'eau (24) associé avec l'ouverture (22), le passage au-dessus de bords périphériques correspondants (26, 28) de la première partie (18) et de la seconde partie (20), lorsque le corps en forme de boîte (14) est à l'état ouvert.
